# EUROPEAN PATENT SPECIFICATION

(11) **EP 3 561 064 B1**
(45) Date of publication and mention of the grant of the patent: **14.02.2024**
(21) Application number: 18169907.5
(22) Date of filing: 27.04.2018
(51) Int. Cl.: C12P 7/06, C12M 1/34

(54) **PROCESS AND SYSTEM FOR MICROBIAL FERMENTATION**
VERFAHREN UND SYSTEM ZUR MIKROBIELLEN FERMENTATION
PROCÉDÉ ET SYSTÈME DE FERMENTATION MICROBIENNE

(43) Date of publication of application: 30.10.2019
(73) Proprietor: Sekab E-Technology AB, 891 26 Örnsköldsvik (SE); Terranol A/S, 2450 København SV (DK)
(72) Inventor: CAVKA, Adnan, SE-891 96 ARNÄSVALL (SE); WENNBERG, Patrik, SE-891 92 ÖRNSKÖLDSVIK (SE); ANDERSEN, Thomas Hvid, DK-2000 FREDERIKSBERG (DK); KNUDSEN, Jan Dines, SE-212 18 MALMÖ (SE); RØNNOW, Birgitte, DK-1267 KØBENHAVN K (DK); SIBBESEN, Ole, DK-2880 BAGSVAERD (DK)
(74) Representative: Kransell & Wennborg KB

(56) References cited:
- WO-A1-2012/066042
- RØNNOW: "Bioenergy - from research to market deployment in a European context", CoRyFee Demonstration Project; Stockholm, June 14th, 2017 Conference presentation, 2017, pages 1-12, XP002785168, Retrieved from the Internet: URL:http://eranetbestf.net/wp-content/uplo ads/2017/11/Terranol.pdf [retrieved on 2018-09-26]
- VAN LEEUWEN-JONES ET AL (EDITORS): "Bioenergy: from research to market deployment in a European context", BESTF PROGRAMME Project presentation pamphlet, 2017, pages 1-16, XP002785170, Retrieved from the Internet: URL:http://www.bioprogress.se/resources/BE STF_brochure.pdf [retrieved on 2018-09-26]
- KNUDSEN ET AL: "Reducing the fermentation costs for lignocellulosic ethanol production by closing the gap between fed-batch and continuous fermentation", 40th Symposium on Biotechnology for Fuels and Chemicals Abstract of a symposium paper, 1 May 2018 (2018-05-01), page 1, XP002785169, Retrieved from the Internet: URL:https://sim.confex.com/sim/40th/meetin gapp.cgi/Paper/36406 [retrieved on 2018-09-26]
- MITHRA ET AL: "Strategies for enzyme saving during saccharification of pretreated lignocellulo-starch biomass: effect of enzyme dosage and detoxification chemicals", HELIYON, vol. 3, 2017, pages 1-22, XP002785183,
- NILSSON A. ET AL: "On-line estimation of sugar concentration for control of fed-batch fermentation of lignocellulosic hydrolyzates by Saccharomyces cerevisiae", BIOPROCESS AND BIOSYSTEMS ENGINEERING, vol. 25, no. 3, 9 August 2002 (2002-08-09) , pages 183-191, XP55735892, DE ISSN: 1615-7591, DOI: 10.1007/s00449-002-0293-x
- Wesley Thompson ET AL: "Real time analysis of biomass fermentation using Raman spectroscopy", , 23 March 2011 (2011-03-23), XP55735814, Retrieved from the Internet: URL:https://depts.washington.edu/cpac/Acti vities/Meetings/Satellite/2011/Presentatio ns/Wednesday/4%20-%20Thompson%20Raman%20fo r%20Fermentation.pdf

## Description

### TECHNICAL FIELD

The present invention generally relates to microbial fermentation and in particular to a method for control of a microbial fermentation process involving co-fermentation of sugars from lignocellulosic biomass to fermentation products.

### BACKGROUND

Global warming, petroleum depletion and energy security have been the main driving forces for the development of renewable fuels that can replace the petroleum-derived fuels, such as gasoline and diesel. Microbially produced ethanol (often referred to as *bioethanol*) is currently the most commonly used renewable automobile fuel. It is largely produced by fermentation of sugars derived from cellulosic biomass, such as sugar- or starch-containing feedstocks, e.g. cane sugar, corn and wheat. However, the supply of these crops is relatively limited, and many of them can be considered as a human food resource. Cellulosic biomass comprising lignin (*lignocellulosic biomass*, also referred to simply as *lignocellulose*)*,* on the other hand, is a more abundant and less expensive raw material, often considered as waste, with the potential to give a high net energy gain.

Lignocellulose is composed of cellulose, hemicellulose and lignin. Cellulose is composed of polysaccharide chains of several hundred to over ten thousand linked glucose units, whereas hemicellulose is a polysaccharide composed of xylose, other pentose sugars and various hexose sugars. In lignocellulose, cellulose and hemicellulose are tightly associated to lignin, a polyphenolic compound that ties the cellulose and hemicellulose polymers together, thus providing the lignocellulose with rigidity and mechanical strength.

In the production of ethanol from lignocellulosic materials, various pretreatment and hydrolysis steps are typically used to degrade the cellulose and hemicellulose polysaccharides in the lignocellulose to fermentable saccharides. The fermentable saccharides are then converted to ethanol by fermentation with microorganisms such as yeast or bacteria, and the ethanol is recovered by means of distillation. The yeast *Saccharomyces cerevisiae*, for example, metabolizes hexose sugars and is a microorganism suitable for industrial processes for cellulosic bioethanol production.

Nilsson A. et al. (2002) On-line estimation of sugar concentration for control of fed-batch fermentation of lignocellulosic hydrolyzates by Saccharomyces cerevisiae. BIOPROCESS AND BIOSYSTEMS ENGINEERING 25(3) :183-191, describes a method for the control of a microbial fermentation process involving co-fermentation of sugars from lignocellulosic biomass.

Bioethanol production by fermentation of hydrolyzed residual lignocellulosic biomass has the potential of up to 85% reduction of CO₂-emissions as compared to gasoline. However, in order to be more commercially interesting, there are still improvements to be made, including improvements of the microbial fermentation stage. Increased yield and cost reductions are continuously strived for.

### SUMMARY

A general object of the present invention is to provide an improved microbial fermentation process. A specific object is to provide an improved microbial fermentation process suitable for fermentation of lignocellulosic biomass materials to fermentation products, such as ethanol. Another object is to provide a more efficient microbial fermentation process resulting in yield improvements and/or cost reductions.

These objects are achieved in accordance with the attached claims.

Briefly, efficient microbial co-fermentation of cellulose and hemicellulose derived sugars originating from lignocellulosic biomass is achieved by automatic feed control wherein a continuous (or semi-continuous) flow of sugars into the fermentation vessel is adapted in response to a residual sugar indicator indicating the concentration of residual sugars in the fermentation vessel. The rate of change of the residual sugar indicator is determined and used for controlling the feed of sugars so as to achieve and maintain optimum/maximum fermentation rate. In this way, the present invention provides for optimized fermentation conditions and enables a favorable steady state condition to be reached in the fermentation. The steady state condition is associated with in situ propagation of microorganisms, which surprisingly has proved to render additional inoculum unnecessary in, for example, repeated fed-batch fermentation controlled in accordance with the invention. Preferably, the optimum/maximum fermentation rate is used to detect and maintain the steady state condition.

More specifically, a method for control of a microbial fermentation process involving co-fermentation of sugars from lignocellulosic biomass to fermentation products by means of fermentation microorganisms is provided. The method comprises continuous or semi-continuous addition, to a fermentation vessel, of a fermentation media comprising at least two different sugars; providing an initial active population of the fermentation microorganisms into the fermentation vessel; online measuring of a residual sugar indicator parameter RSI, which parameter directly or indirectly indicates the concentration of residual sugars, during fermentation in the fermentation vessel; determining an RSI setpoint based on a rate of change of the measured residual sugar indicator parameter, such that the RSI setpoint corresponds to a maximum rate of change; and automatically adapting the amount of sugar added to the fermentation vessel in a predetermined manner in response to the measured residual sugar indicator parameter RSI and the RSI setpoint, so as to achieve and maintain the RSI setpoint, whereby efficient co-fermentation of sugars to fermentation products is obtained.

According to an advantageous embodiment, the RSI setpoint corresponds to a steady state condition in the fermentation vessel and the method further comprises in situ growing of the fermentation microorganisms in the fermentation vessel by means of the sugars added to the fermentation vessel, so as to maintain a steady-state population of fermentation microorganisms in the fermentation vessel, whereby further addition of microorganisms is not required.

Advantages associated with the present invention, are inter alia:
- more efficient co-fermentation
- utilization of a higher proportion of the sugars available
- yield improvement
- increased volumetric productivity (cost reduction)
- reduced operational costs, e.g. yeast cost reduction due to in situ propagation
- cost reduction in yeast fermentation for commercial production of cellulosic ethanol

The RSI setpoint may, according to one embodiment, comprise a target interval of the RSI or a target interval of the RSI rate of change, and the step of automatically adapting in turn comprises the steps of: comparing measured, and preferably processed, values of the residual sugar indicator parameter to the RSI setpoint; and adjusting the amount of sugar added to the fermentation vessel in response to the comparison, so as to reach and stay within the RSI setpoint.

The fermentation process may, according to one embodiment, be a fed-batch fermentation process comprising at least one batch phase, feed phase and end phase, respectively, and having continuous addition of fermentation media during the at least one feed phase.

The fed-batch fermentation process may, according to one embodiment, be a repeated fed-batch fermentation process with continuous addition of fermentation media during at least two feed phases.

The respective feed phase or feed phases of the fermentation process may, according to one embodiment, be extended so as to comprise a substantial portion of the fed-batch cycle.

The fermentation process may, according to one embodiment, be a fed-batch fermentation process wherein the step of determining an RSI setpoint is based on initial RSI measurements during the batch phase, and the step of automatically adapting comprises automatically adapting, during the feed phase, the amount of sugar added to the fermentation vessel in a predetermined manner in response to RSI measurements during the feed phase and said RSI setpoint, so as to achieve and maintain said RSI setpoint. The step of providing an initial active population is preferably preceded by partially filling the fermentation vessel with fermentation media.

The fermentation process may, according to one embodiment, comprise the step of secondary fermentation, in at least one secondary fermentation vessel arranged downstream of the fermentation vessel, to further increase the co-fermentation of sugars to fermentation products.

The fermentation process may, according to one embodiment, be a fed-batch fermentation process wherein the secondary fermentation comprises directing an overflow of the fermentation vessel into at least one secondary fermentation vessel to finish the fermentation of the residual sugars.

The fermentation process may, according to one embodiment, have a measuring step which involves density measurements.

The measuring step may involve refractive index (RI) measurements and the residual sugar indicator parameter RSI comprises a refractive index (RI) parameter.

The measuring step may involve any of a combination of measurements selected from the group of: optical measurements within UV, visual or IR wavelengths; measurements of carbon dioxide (CO₂) generation; and direct measurements of sugar concentration preferably using chromatography, sugar assay kits, or glucometers.

The fermentation process may, according to one embodiment, comprise addition, to the fermentation vessel, of at least two separate sugar streams associated with the respective different sugars; and individual adjustment of the respective at least two separate sugar streams associated with the respective different sugars.

The fermentation process may, according to one embodiment, comprise recirculation of fermentation microorganisms from a position downstream of the fermentation vessel and back into the fermentation vessel.

According to an example aspect not covered by the claims, a system for control of a microbial fermentation process is provided.

More specifically, a system for control of a microbial fermentation process involving co-fermentation, in a fermentation vessel, of sugars from lignocellulosic biomass to fermentation products by means of fermentation microorganisms is provided. The system comprises means for continuous or semi-continuous addition, to the fermentation vessel, of a fermentation media comprising at least two different sugars; measuring means for online measuring of a residual sugar indicator parameter RSI, which parameter directly or indirectly indicates the concentration of residual sugars, during fermentation in the fermentation vessel; means for determining and setting an RSI setpoint based on a rate of change of the measured residual sugar indicator parameter, such that the RSI setpoint corresponds to a maximum rate of change; and control means for automatically adapting the amount of sugar added to the fermentation vessel in a predetermined manner in response to the measured residual sugar indicator parameter RSI and the RSI setpoint, so as to achieve and maintain the RSI setpoint, whereby efficient co-fermentation of sugars to fermentation products is obtained.

According to a preferred example not covered by the claims, the system further comprises means for in situ growing of the fermentation microorganisms in the fermentation vessel by means of the sugars added to the fermentation vessel, so as to maintain a steady-state population of fermentation microorganisms in the fermentation vessel, the RSI setpoint corresponding to the steady state condition in the fermentation vessel.

According to one example not covered by the claims, the RSI setpoint comprises a target interval of the RSI or a target interval of the RSI rate of change, and the control means for automatically adapting in turn comprises: means for comparing measured, and preferably processed, values of the residual sugar indicator parameter to the RSI setpoint; and means for adjusting the amount of sugar added to the fermentation vessel in response to the comparison, so as to reach and stay within the RSI setpoint.

According to one example not covered by the claims, the system is a fed-batch fermentation system arranged for fed-batch fermentation in at least one batch phase, feed phase and end phase, respectively, with continuous addition of fermentation media during the at least one feed phase.

According to one example not covered by the claims, the system comprises a secondary fermentation unit arranged downstream of the fermentation vessel, for further increased co-fermentation of sugars to fermentation products, preferably in at least two secondary fermentation vessels connected in series and/or at least two secondary fermentation vessels connected in parallel.

According to one example not covered by the claims, the system comprises refractive index (RI) measurement means.

According to one example not covered by the claims, the system comprises means for separate addition, to the fermentation vessel, of at least two separate sugar streams associated with the respective different sugars; and means for individual adjustment of the respective at least two separate sugar streams associated with the respective different sugars.

According to one example not covered by the claims, the system comprises means for recirculation of fermentation microorganisms from a position downstream of the fermentation vessel and back into the fermentation vessel.

According to yet another example aspect not covered by the claims, a system for producing fermentation products, preferably including ethanol, from lignocellulosic biomass is provided.

### BRIEF DESCRIPTION OF THE DRAWINGS

The invention, together with further objects and advantages thereof, is best understood by reference to the following description and the accompanying drawings, in which:
- Fig. 1: is a schematic view of a system for producing ethanol from lignocellulosic biomass according to one example;
- Fig. 2: is a schematic view of a fermentation control system according to one example;
- Fig. 3: is a flow chart of an exemplary embodiment of the method for fermentation control according to the present invention;
- Fig. 4: is a flow chart of another exemplary embodiment of the method for fermentation control according to the present invention;
- Fig. 5: is a diagram illustrating refractive index (RI) and residual sugars during fermentation with automatic feed control according to an exemplary embodiment of the present invention;
- Fig. 6: is a diagram illustrating fermentation with automatic feed control according to another exemplary embodiment of the present invention;
- Fig. 7: is a diagram illustrating fermentation with automatic feed control according to another exemplary embodiment of the present invention with secondary fermentation; and
- Fig. 8: A and B are schematic drawings illustrating secondary fermentation configuration with serial and parallel mode according to exemplary embodiments of the present invention.

### DETAILED DESCRIPTION

Throughout the drawings the same reference numbers are used for similar or corresponding elements.

The term *microbial fermentation* herein refers to fermentation by microorganisms. The term *co-fermentation* herein refers to the simultaneous fermentation of two (or more) different sugars in the same fermentation reactor / process step.

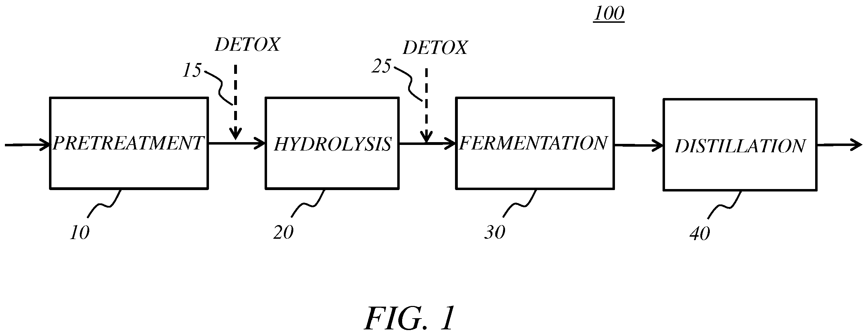

Fig. 1 is a schematic view of a system 100 for producing ethanol from lignocellulosic biomass according to one example. In the illustrated example, input raw material or source material is processed in a pretreatment unit 10 and a hydrolysis unit 20 before the hydrolysate enters a fermentation unit/system 30. Various pretreatment and hydrolysis units/systems known in the art can be used to degrade the polysaccharides in the lignocellulose to fermentable sugars. The pretreatment unit 10 may for instance also include an impregnation unit.

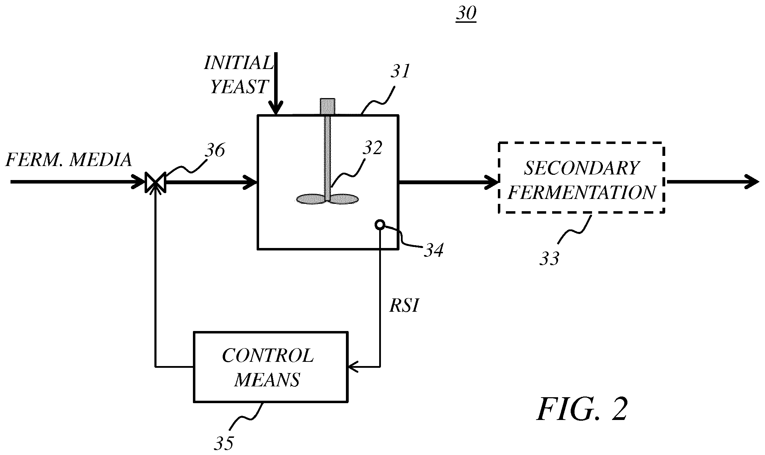

The fermentation unit/system 30 comprises one or more fermentation vessels/reactors (31 in Fig. 2) in which two or more fermentable sugars are converted to ethanol by fermentation with microorganisms. The ethanol is recovered by means of distillation in a distillation unit/ system 40 connected to the fermentation unit 30.

Preferred embodiments of the present invention have separate hydrolysis and fermentation units (SHF) 20, 30, which facilitates the feed control. However, under certain circumstances it may also be possible to use the inventive feed control in systems with simultaneous saccharification and fermentation (SSF), i.e. hydrolysis and fermentation in the same fermentation reactor.

In Fig. 1 and other herein described embodiments, the primary fermentation product is exemplified as ethanol. However, it should be understood that the present invention is also applicable to processes resulting in other fermentation products, such as other alcohols, organic acids and fatty acids. According to one advantageous embodiment the fermentation product is lactic acid.

The source material in the process and system of the present invention may be any lignocellulosic biomass, including softwood and hardwood. Fermentation media comprising lignocellulosic biomass or thereof derived sugars is input to the fermentation system, where sugars from the lignocellulosic biomass input material are co-fermented. The fermentation media may often be a hydrolysate from a preceding hydrolysis stage.

As illustrated by dashed arrows in Fig. 1, there may be an optional detoxification 15 between the pretreatment and hydrolysis units 10, 20 and/or an optional detoxification 25 between the hydrolysis and fermentation units 20, 30. Using such a detoxified hydrolysate as fermentation media has advantages in connection with the present invention, as will be described in the following.

Fig. 2 is a schematic view of a system 30 for controlled fermentation according to one example. The system 30 comprises a fermentation vessel (i.e. fermentation reactor) 31 which receives a fermentation media, such as a hydrolysate, comprising at least two different sugars. In the fermentation vessel 31, co-fermentation of different sugars is achieved by means of suitable conventional co-fermentation microorganisms, such as *Saccharomyces cerevisiae* fermentation yeasts suitable for ethanol production from lignocellulosic biomass and modified by metabolic engineering. Yeast strains commercially available from the company Terranol A/S, including strain V 1, cv-40 and cv-110, may for example be used. The fermentation vessel 31 has agitation means 32, e.g. a conventional rotor with associated motor. The agitation means 32 is preferably arranged so as to achieve mixing throughout substantially the entire fermentation vessel 31, to facilitate the contact between the microorganisms and fermentation media.

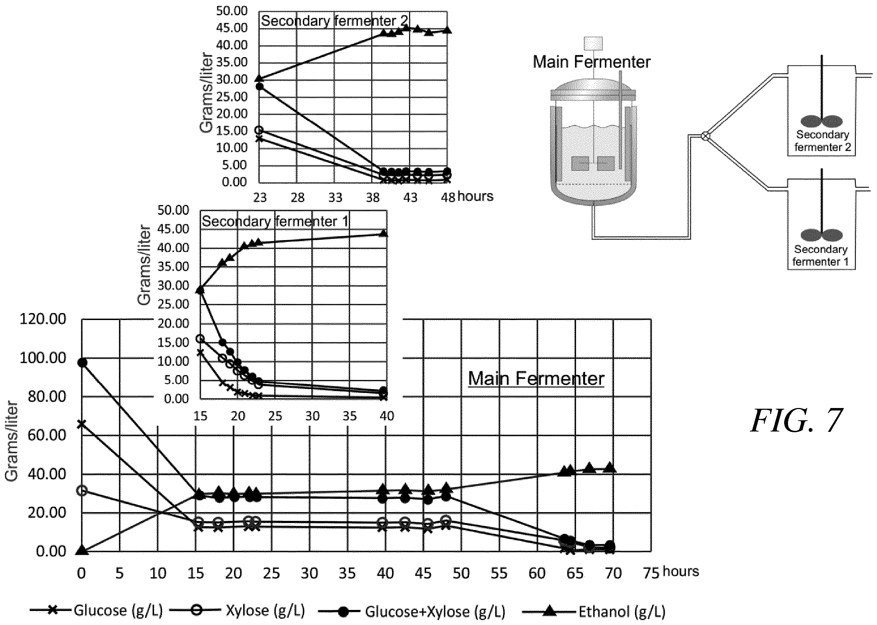

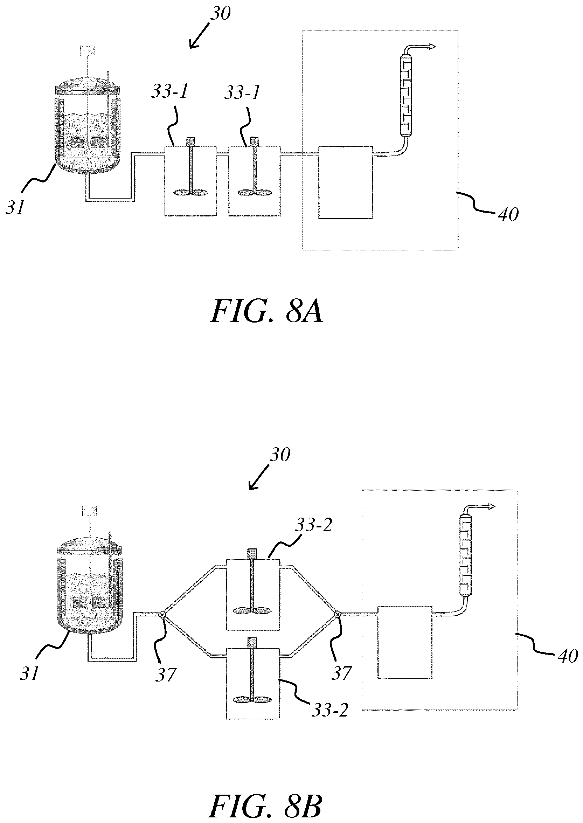

Downstream the fermentation vessel 31, there is an optional secondary fermentation stage 33 for further fermentation of sugars to fermentation products. (Preferred embodiments of the secondary fermentation are illustrated in Figs. 7 and 8 and further described below.)

The fermentation control system 30 further comprises means 34, 35 for measuring of a residual sugar indicator parameter RSI, which directly or indirectly indicates the concentration of residual sugars in the fermentation vessel 31. The measuring means typically includes one or more sensors 34 arranged in the reactor (Fig. 2) or in a loop outside the reactor (for example using a recirculation pump). There may also be embodiments with sensors in the reactor outflow (of output liquid fermentation products or output CO₂). Sensors can in addition also be arranged in the reactor inflow, providing RSI signals used in combination with RSI measured in the outflow.

Both inline and online RSI measurements are thus possible.

The measured RSI signal is received by control means 35, which may e.g. be a computer control means designed to automatically adapt the amount of sugar added to the fermentation vessel 31 in a predetermined manner in response to the measured RSI signal. In the example of Fig. 2, the control means 35 communicates with a valve 36 so as to achieve the variable fermentation media stream input to the fermentation vessel 31, but the skilled person understands that other equipment and process designs are possible.

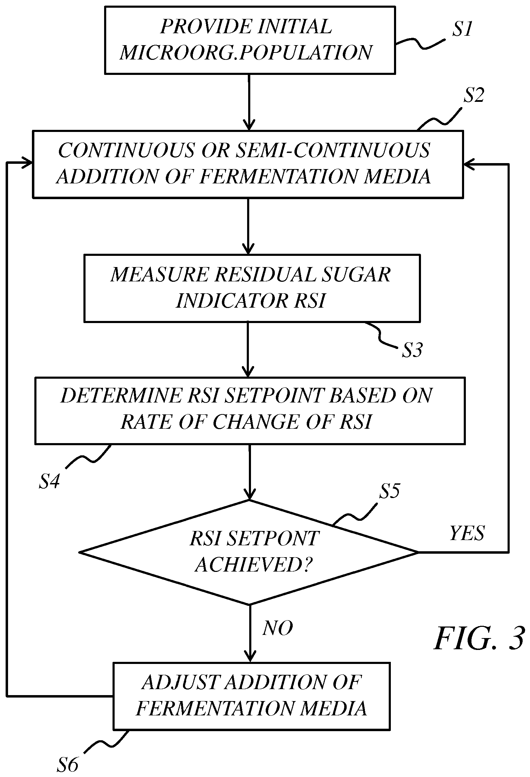

Fig. 3 is a flow chart of an exemplary embodiment of the method for control of a microbial fermentation process involving co-fermentation of sugars from lignocellulosic biomass according to the present invention. In step S1, an initial population of fermentation microorganisms is provided, by addition into the fermentation vessel. A fermentation media comprising at least two different sugars is continuously added to the fermentation vessel (step S2). The addition of fermentation media is performed as a continuous feed for a substantial time, in continuous or semi-continuous operation.

At the point when the initial microorganism population is added into the fermentation vessel, the fermentation vessel would typically be containing, or receiving, an amount of fermentation media.

In step S3, a residual sugar indicator parameter RSI, which directly or indirectly indicates the concentration of residual sugars, during fermentation in the fermentation vessel, is measured. The RSI may be a density indicator, for example obtained using optical measurements or refractive index (RI) measurements. The RSI could also be derived from measurements and calculations of carbon dioxide (CO₂) generation, or obtained by direct measurements of sugar concentration.

An *RSI setpoint* is in step S4 determined based on a rate of change of the measured values of the residual sugar indicator parameter RSI, such that the RSI setpoint corresponds to a maximum or optional rate of change. The RSI setpoint is a target parameter (also referred to as desired parameter or reference parameter). It may be a target value or an interval corresponding to desirable or optimum process conditions. The RSI setpoint may be fixed or variable. The determining of the RSI setpoint may involve calculations and/or online following of the registered RSI signal during the fermentation process. In a preferred embodiment, the RSI setpoint corresponds to a steady state condition in the fermentation vessel.

According to the invention, the amount of sugar added to the fermentation vessel is automatically adapted in a predetermined manner in response to the measured residual sugar indicator parameter RSI and the RSI setpoint, so as to achieve and maintain the RSI setpoint, whereby efficient co-fermentation of sugars to fermentation products is obtained.

Step S5 asks if the RSI setpoint is achieved. This could mean comparing the measured, and possibly processed, RSI values (registered actual RSI) to the RSI setpoint. It could for example also mean comparing calculated derivative values of the registered RSI to a RSI setpoint defined as the maximum or optional rate of change of RSI. If the desired interval is not met, the amount of sugar added to the fermentation vessel is adjusted in response to the comparison, so as to achieve (or come closer to) a desired fermentation rate (step S6). If, on the other hand, the RSI setpoint is reached, i.e. the rate of change is within the desired interval, the fermentation media addition can continue without adjustment and the process returns to step S2.

The present invention refers to fermentation with continuous feed of fermentation media for the whole or part of the process running time (i.e. for at least a portion of the process running time). In other words, the addition of the fermentation media to the fermentation vessel according to the present invention may be continuous or semi-continuous.

The operation of the process may be *continuous* with continuous addition of fermentation media during substantially the whole process running time.

The operation of the process may for example be prolonged into a continuous phase after completed filling of the fermentation vessel by controlled feed, by continuous controlled or continuous addition of fermentation media.

The operation of the process is preferably *fed-batch* or *repeated fed-batch*, with continuous addition of fermentation media during the at least one feed phase.

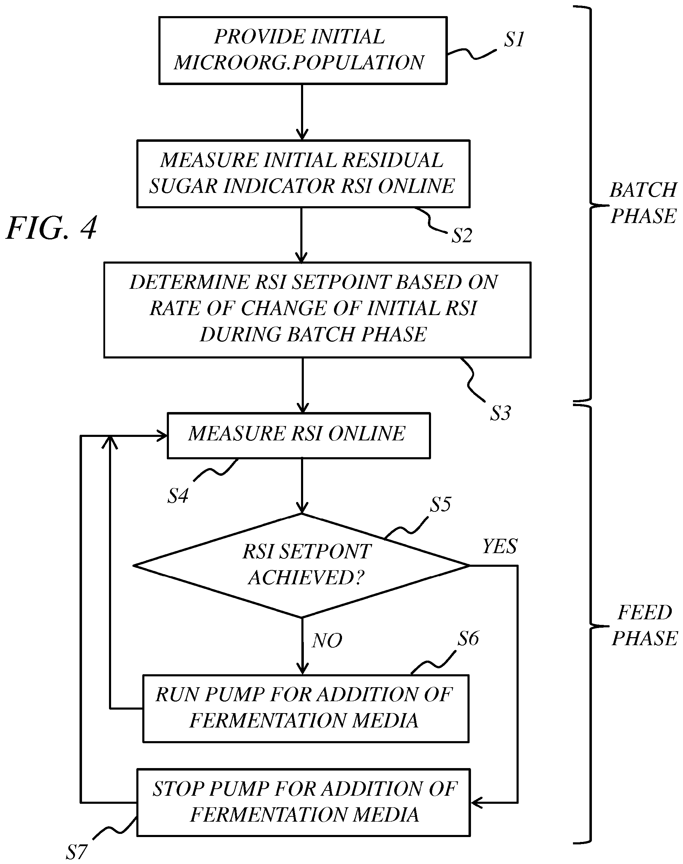

Fig. 4 is a flow chart illustrating an exemplary embodiment of the present invention, with automatic control of a fed-batch fermentation process. In step S1, an initial population of fermentation microorganisms is added into a fermentation vessel containing an amount of lignocellulosic biomass hydrolysate comprising at least two different sugars.

In step S2, a residual sugar indicator parameter RSI, which directly or indirectly indicates the concentration of residual sugars, is measured at the onset of the fermentation in the fermentation vessel. The RSI may be a density indicator, for example obtained using optical measurements or refractive index (RI) measurements. The RSI could also be derived from measurements and calculations of carbon dioxide (CO₂) generation, or obtained by direct measurement of sugar concentration.

In step S3 the RSI setpoint is determined by calculation based on initial RSI measurements or by another determination based on online measured RIS during the batch phase of the fermentation. In step S4, the continuous online measurement of the RSI parameter proceeds after the RSI setpoint has been inserted or set. In step S5, the measured RSI value is compared with the RSI setpoint. Addition of a fermentation media comprising at least two different sugars is set in run state (S6) or in stop state (S7) according to the result in S5. The measurement in S4, the decision in S5 and the running or stopping of the pump is repeated with preset fixed intervals that may be measured in seconds, minutes or hours. Overflow from the fermentation vessel is preferably collected in a secondary fermentation stage to complete the fermentation.

In accordance with the present invention, a *residual sugar indicator parameter RSI* is measured and used to control the fermentation. RSI directly or indirectly indicates the concentration of residual sugars, i.e. the remaining sugars (individual or total) during the microbial fermentation in the fermentation vessel. RSI is used as a control parameter in the automatic feed control.

In some embodiments, RSI is a density indicator, obtained from online density measurements of the content of the fermentation vessel. Refractive index (RI) measurements may for example be used. Optical measurements within UV, visual or IR wavelengths may also be used.

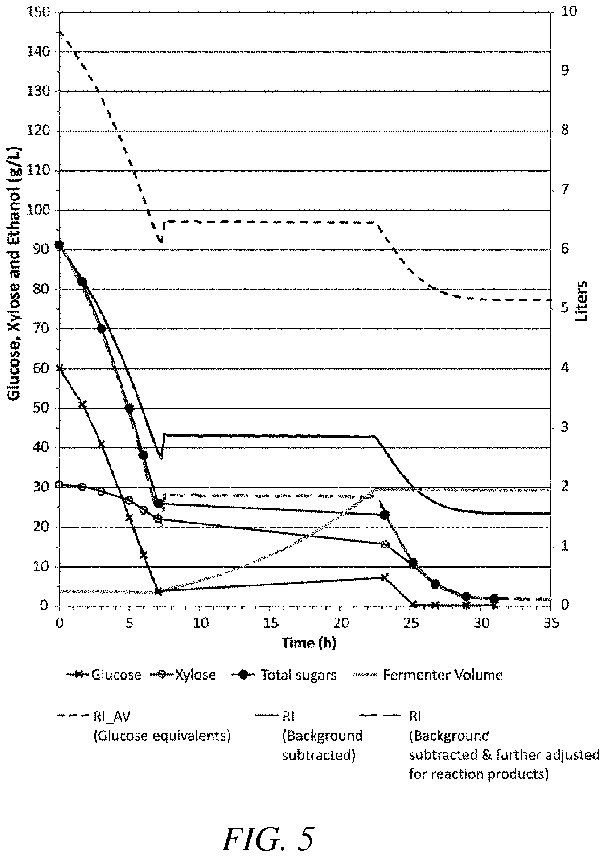

A preferred embodiment with RI (refractive index) controlled feeding is illustrated by the example diagram of Fig. 5, which relates to fed-batch fermentation with online RI monitoring and control. Residual sugars (glucose, xylose, and total sugars) determined by HPLC and RI (in glucose equivalents) are shown. The comparatively simple RI measurements have proven to reflect the sugar concentrations from HPLC surprisingly well. Fig. 5 implies that RI in particular is very useful as residual sugar indicator RSI for the automatic feed control in accordance with the present invention.

With processing of the output RI signal (adjusting for background and relating the signal output with for instance HPLC data) it can be directly correlated to that of residual sugars. The inventors have shown that it is possible to calculate residual fermentable sugars from online RI during the fermentation through this method with high accuracy.

Thus, online RI measurements can be used either unprocessed or processed to estimate/calculate residual sugar content, for fast and efficient co-fermentation control in accordance with preferred embodiments of the present invention.

RI is preferably measured online in the fermenter, or in a looped tube circulating fermentation broth in the fermenter, or in the outlet tube. RI is preferably measured with a suitable refractometer.

Process conditions of the Fig. 5 example: *Lignocellulosic (wheat straw) hydrolysate with solids. Yeast strain cV-110 (Terranol), 0.5 g*/*L total pitch (4 g*/*L initially in batch), pH 5.2, 32°C*

Indirect residual sugar indicators RSI may also be used, for example obtained from carbon dioxide (CO₂) measurements. CO₂ can then be measured as such with CO₂ gas sensors, or, alternatively, be indirectly measured preferably via gas pressure P or via the degree of opening of the CO₂ exhaust valve.

The use of CO₂ generation as the RSI parameter would typically require an online calculation to transform the total generated CO₂ amount into an amount of fermented sugar and the corresponding change of concentration of sugars in the fermentation vessel.

It would even be possible to use the ethanol output from the fermentation process as indirect residual sugar indicator RSI. The sugar content is then derived from the well-known relationship between sugar, CO2 and ethanol.

There are also embodiments of the invention which use direct measurements of sugar concentration, preferably using chromatography, sugar assay kits, or glucometers.

For increased control accuracy and process efficiency, there are also embodiments of the invention which use a *combination* of measurements, for example density and CO₂ measurements, or density, HPLC and CO₂ measurements.

The registered signal of the residual sugar indicator parameter RSI is generally processed using a mathematical function, such as a moving average and/or derivate of the residual sugar indicator.

The desired parameter value/interval, RSI setpoint, may be changed during operation, manually or automatically. It may also be set to be dependent of, i.e. as a mathematical function of, one or more other parameters.

By means of the fermentation control according to the present invention, efficient co-fermentation of sugars from lignocellulosic biomass can be achieved. Adapting the feed of fermentation media, e.g. hydrolysate, into the fermentation vessel in response to the registered residual sugar indicator RSI, leads to a more efficient use of the sugars available and utilizing of a higher proportion of the sugars available. This means yield improvements by increased fermentation efficiency and also cost reductions due to increased volumetric productivity. The invention also enables reduced operational costs (including reduced costs for yeast).

According to a preferred embodiment of the invention the feed of fermentation media is adapted so as to optimize the fermentation rate. This is based on the insight that an optimum fermentation rate corresponds to a steady state condition of the microbial fermentation. In the steady state condition, RSI may thus be maintained at substantially the same level despite continuous addition of fermentation media and no addition of microorganisms.

The inventors have unexpectedly found that, after an initial population of microorganisms, typically yeast, is provided, additional inoculum is not required during fermentation controlled in accordance with the present invention. The hypothesis was that, due to dilution of the yeast, additional inoculum (external supply) would be needed between phases in repeated fed-batch operation, for example. However, it was surprisingly discovered that the yeast concentration is maintained. The fermentation vessel can be emptied until only about 20-25% of the volume remains, and still no refill of yeast is needed since the yeast is, by means of the at least partially continuous stream of sugar added, growing with increased volume in the fermentation vessel.

Basically, the microorganisms (e.g. yeast) will, provided that they have sufficient sugar, grow until an optimum/maximum rate of fermentation is achieved. This optimum fermentation rate is detected via the RSI measurements in the feed controlled system according to the present invention. At the optimum fermentation rate a steady-state condition is achieved by in situ propagation.

The process of the invention preferably comprises achieving in-situ propagation of fermentation microorganisms resulting in a steady-state population so that additional inoculum is not required during fermentation. This means that the added amount of sugar is adapted so as to achieve and maintain the optimum fermentation rate and hence the steady-state population of microorganisms.

The desirable steady state condition would in accordance with a preferred embodiment of the invention be detected via determining the optimum, i.e. typically the maximum, fermentation rate of the process from RSI measurements. The steady state condition is achieved at an RSI that is the same at least for a substantial period of time.

The amount of sugars available would in general be decisive for the yeast growth (in situ propagation) and the desirable steady-state condition could also be expressed as an optimum yeast to sugar ratio, e.g. determined from online measurements of sugar and yeast concentration.

The system will automatically reach a new steady state in response to changed conditions. If, for example, the ratio sugar:yeast is altered by manually lowering or increasing the sugar concentration, the yeast will adjust, by either growing further to a new steady state or, in case of shortage of sugar in relation to yeast, some starvation in the population will occur, reaching a new steady state with lower yeast concentration.

Fig. 5, 6 and 7 are diagrams illustrating fed-batch fermentation with automatic feed control according to exemplary embodiments of the present invention.

Fig. 5 illustrates *one phase* fed-batch fermentation of wheat straw derived lignocellulosic material with automatic feed control according to the invention. The volume of fermentation media in the fermentation vessel during the respective batch, feed and end phases is shown. (The volume increase indirectly illustrates the addition of fermentation media.) Sugar content (glucose and xylose, in this example) is shown. RI (refractive index) is used as residual sugar indicator, i.e. the feed control is performed using online RI measurements as input signal.

It is clear from Fig. 5 that RI is very suitable for indicating residual sugars during fermentation with automatic feed control according to the present invention.

The one phase fed-batch process of Fig. 5 has a comparatively short feed phase (about 15h, or about 50% of the fed-batch cycle). As shown by Fig 5, low content of residual sugars, i.e. efficient fermentation, is still achieved with one phase fed-batch fermentation controlled in accordance with the invention. A high ethanol yield (> 90%) was obtained.

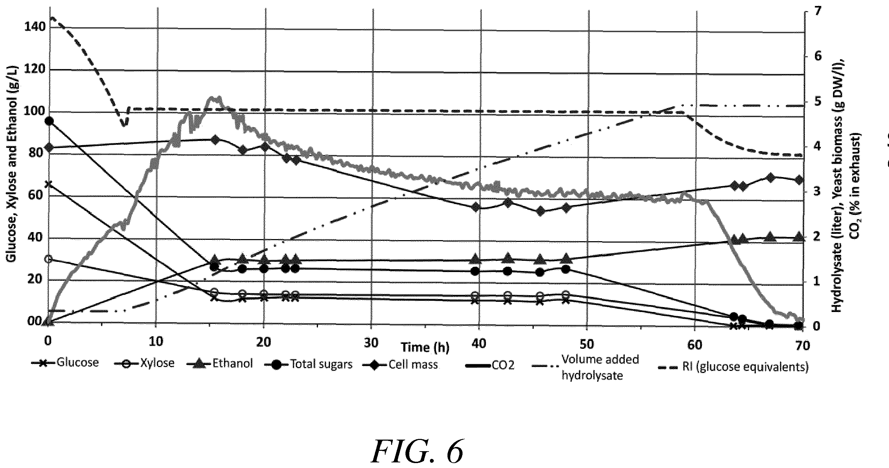

Fig. 6 illustrates fed-batch fermentation of lignocellulosic material with automatic feed control according to another embodiment of the invention, using an extended (also referred to as prolonged or continued) feed phase.

The lignocellulosic material used as fermentation media is in this example wheat straw hydrolysate. The wheat straw hydrolysate substrate was supplied with urea as nitrogen source (3 g/1) and adjusted to pH 5.5. The same hydrolysate was used for the batch phase and the feed phase.

The batch phase (25 % of full fermenter filling of 1 liter) was inoculated with 3.9 gram/liter DW of yeast strain cV-110.

The volume of fermentation media in the fermentation vessel during the respective batch, feed and end phases is shown. (The volume increase indirectly illustrates the addition of fermentation media.) Sugar content (glucose and xylose, in this example) is shown, as well as the fermentation products ethanol and CO₂. RI is used as residual sugar indicator, i.e. the feed control is performed using online RI measurements as input signal.

When the RI value corresponding to the highest RI change rate was found, this was used as setpoint throughout the rest of the feed phase. The pH was maintained and controlled using dilute sulphuric acid and 20 % ammonia solution, and the temperature kept at 32°C.

As illustrated by Fig. 6, the continued fed-batch fermentation process with automatic feed control of the invention results in a high ethanol yield (>90%) and low content of residual sugars. Both glucose and xylose were consumed by the fermentation microorganisms to extremely low levels.

The one phase fed-batch process of Fig. 6 has a comparatively long feed phase (about 45h, or about 65% of the illustrated fed-batch cycle). This is advantageous inter alia due to the fact that the ethanol productivity (g ethanol per g yeast and time) is higher during the feed phase than during the batch phase and end phase.

In a process with extended feed phase(s), the respective feed phase or feed phases of the fermentation process would be extended a certain time beyond the point where the fermenter is full, by continuing the feeding and removing the surplus overflow of fermentation broth from the fermenter. An extended feed phase preferably has a length corresponding to at least 60%, more preferably at least 70%, of the fed-batch cycle.

As mentioned earlier in connection with Fig. 2, preferred embodiments of the present invention may also include *secondary fermentation*, in at least one secondary fermentation vessel 33 arranged downstream of the fermentation vessel 31, to further increase the co-fermentation of sugars to fermentation products.

In the example illustrated by Fig. 7, the fermentation process of Fig. 6, in a main fermenter, is combined with a secondary fermentation process in secondary fermenters.

Upon continued fed-batch fermentation of lignocellulosic material with automatic feed control according to Fig. 6, the overflow of the main fermenter was directed into two parallel connected secondary fermenters. The feeding was continued for several additional fermenter volumes and the overflow directed into a waste container, which showed that at the late phase at 48 hours and forwardly a steady state of CO₂ generation is obtained.

The secondary tanks were stirred and thermostated at 30°C, without any other controls or adjustments. If the secondary tanks are sufficiently insulated, only stirring is needed.

Feeding to the main fermenter was stopped after feeding of 5 fermenter volumes (5 liter, at 58 hours) and the fermenter stirring remained until the fermentation was finished after approximately 68 hours.

Continued (i.e. extended) fed-batch, as in Fig. 6 and 7, results in a higher ethanol yield as higher yield is obtained in the feed phase and end phase compared to the batch phase. A further advantage is that the prolonged feed phase when used with the feed control in accordance with the present invention, results in increased cost-efficiency due to a more efficient use of the microorganisms (typically yeast).

Due to in situ propagation the same initial population of microorganisms can be used for an extended feed phase. This results in reduced yeast expenditure, and consequently in lower operational costs. Fig. 6 clearly illustrates that the fermentation efficiency can be maintained in a steady state with no yeast refill.

Fig. 7 shows that secondary fermentation in at least two secondary fermentation vessels provides for efficient handling of the prolonged feed phase and leads to improved fermentation results. High ethanol yield (>90%) and extremely low levels of residual sugars are achieved.

Similar advantageous effects as with the prolonged feed phase have been observed by the inventors during experiments with *repeated fed-batch* operation with continuous addition of fermentation media during at least two feed phases and wherein the fermentation vessel is only partially emptied between the respective feed phases.

Repeated fed-batch (diagram not shown) results in a high ethanol yield and low content of residual sugars. A further advantage is that two or more consecutive feed phases in the inventive fermentation with feed control result in increased cost-efficiency due to a more efficient use of the microorganisms (typically yeast).

Due to in situ propagation the same initial population of microorganisms can be used again and again. Two or more feed phases without emptying the fermentation vessel in between, result in reduced yeast expenditure, and consequently in lower operational costs. In embodiments of the present invention using repeated feed phase operation, the fermentation efficiency can be maintained with no yeast refill.

The favorable extended, i.e. comparatively long, feed phase can be useful in connection with one phase fed-batch or repeated fed-batch fermentation. As mentioned, such an extended feed phase may be combined with secondary fermentation for efficient handling of the end phase and improved fermentation results.

Secondary fermentation will now be described more in detail with reference to Figs. 7 and 8.

The secondary fermentation is a post-fermentation, typically without further addition of fresh fermentation media or fermentation microorganisms. Sugar remaining after the primary fermentation can thus be consumed in the secondary fermentation. The operation of the secondary fermentation may be batch, fed-batch or continuous.

The secondary fermentation vessel(s) 33 preferably has agitation means, e.g. a conventional rotor with associated motor. The agitation means is preferably arranged so as to achieve mixing throughout substantially the entire fermentation vessel to facilitate the contact between the microorganisms and fermentation media.

Preferably, there are at least two secondary fermentation vessels, connected in series or in parallel. Principles of serial and parallel configurations are schematically illustrated in Fig. 8A and 8B.

Fig. 8A shows a fermentation system 30 with two serially connected secondary fermentation vessels 33-1, arranged downstream of a main fermentation vessel 31 with feed control and upstream of a distillation facility 40. In order to achieve a most efficient fermentation process, the secondary fermentation system is arranged such that there is minimum intermixing between the first and the second secondary fermentation vessel 33-1. One vessel is preferably completely emptied before the filling of the other vessel begins, and so on. In this way, there is time to finish fermentation and thus to reach extremely low levels of residual sugars.

Fig. 8B shows a fermentation system 30 with two parallel connected secondary fermentation vessels 33-2, arranged downstream of a main fermentation vessel 31 with feed control and upstream of a distillation facility 40. In order to achieve a most efficient fermentation process, the secondary fermentation system is arranged such that there is minimum intermixing between the first and the second secondary fermentation vessel 33-2. Flow switching means such as conventional valves 37 are arranged so as to switch back and forth between the two vessels 33-2. This enables fermentation to be completed with extremely low levels of residual sugars. Fig. 8B corresponds to the secondary fermentation set-up used in the process of the example diagrams in Fig. 7.

The number of secondary fermentation vessels 33-1, 33-2 may vary within the scope of the invention and various combinations of serial and parallel vessels are also possible. However, it is preferred to have at least two secondary fermentation vessels, as in Figs. 8A and 8B, and these may with advantage be arranged for interrupted or intermittent flow so as to minimize the mixing between the vessels.

A set up with more than one connected fermentation vessel 33-1, 33-2 in the secondary fermentation is especially useful in combination with fed-batch fermentation or repeated fed-batch fermentation with extended, i.e. comparatively long, feed phase. An extended feed phase may for example be between 24 and 72 hours, its length depending e.g. on raw material, sugar combination, and inhibitor concentration. The connected tanks then take care of the end phase so that the downstream piping, e.g. to the distillation unit, can be of manageable length.

As illustrated by the examples herein (including but not limited to Fig. 5-8), the present invention results in improved fermentation of different sugars, i.e. improved co-fermentation of sugars, from lignocellulosic biomass. The fermentation media input to the fermentation vessel 31 comprises at least two different sugars from lignocellulosic biomass. These are preferably selected from the group of: glucose, mannose, xylose, arabinose, galactose, sucrose and fructose. It should be understood that the primary target molecule can be, for instance, xylose or mannose instead of glucose.

According to another advantageous embodiment of the present invention, the addition of fermentation media involves addition of *at least two separate sugar streams* associated with different sugars. The sugar streams may with advantage be individually adjusted in response to the registered RSI. This further improves the fermentation efficiency.

Separate sugar streams result in improved yield compared to utilizing an initial batch fermentation. As sugar streams are separate this also allows for more flexible fermentation control, i.e. sugars can be added at any rate to create optimal ratios between different sugar streams. (The optimal ratio for glucose:xylose, for example, may not be the same as for glucose: mannose or other combinations.)

Individually adjusted separate streams are also be beneficial for the in situ yeast propagation, since it enables addition of more glucose in relation to the secondary sugar(s) in order to achieve increased biomass formation (microorganism growth), which is sometimes desirable. An excess of glucose, which the microorganisms use for growth, would for instance be favorable if the population over time shows decreased efficiency or performance.

According to another advantageous embodiment of the present invention, the fermentation media added to the fermentation vessel 31 comprises a *detoxified hydrolysate* from preceding detoxification and hydrolysis steps. The detoxification is a treatment/conditioning performed before the fermentation in order to alleviate inhibition problems caused by substances (byproducts) formed during the pretreatment. The detoxification 15, 25 may, as illustrated using dashed arrows in Fig. 1, either be performed before or after the hydrolysis. A detoxification treatment before the hydrolysis would often be preferred when enzymatic hydrolysis is used, since the same byproducts would typically be problematic for both enzymes and fermentation microorganisms. Examples of detoxification methods which may be used include detoxification with reducing agents, overliming/alkali detoxification, ion exchange resins, and activated carbon.

By means of a detoxified hydrolysate, the fermentation control of the present invention becomes even more efficient and cost-effective. The feed phase (of a fed batch process) becomes shorter as compared to when the hydrolysate does not undergo detoxification. Moreover, the lag phase, when the microorganisms to adapt to the growth conditions and get started with the fermentation, is also reduced, which is a further advantage.

According to another advantageous embodiment of the present invention, there is a *recirculation of yeast* back into the fermentation vessel in order to further improve the fermentation efficiency and assist in eliminating the need for yeast refill in a fed-batch system. The yeast is then recirculated from a position downstream the fermentation vessel and back into the inlet or initial portion of the fermentation vessel. In systems with secondary fermentation, this downstream position may be between fermentation vessel and secondary fermentation or immediately after the secondary fermentation. A combination of fed control maintaining a steady state population and recirculation of a portion of the yeast output from the fermentation stage may be used to achieve fast and cost-efficient fermentation. Before recirculation, the yeast would generally need to be separated from other material in the liquid output from the fermentation vessel.

In the illustrated examples, strain cv-110 (available from Terranol A/S)) was used, but other microorganisms suitable for co-fermentation may of course also be used.

## Claims

1. A method for control of a microbial fermentation process involving co-fermentation of sugars from lignocellulosic biomass to fermentation products by means of fermentation microorganisms, comprising the steps of:
continuous or semi-continuous addition, to a fermentation vessel (31), of a fermentation media comprising at least two different sugars;
providing an initial active population of the fermentation microorganisms into the fermentation vessel;
online measuring of a residual sugar indicator parameter RSI, which parameter directly or indirectly indicates the concentration of residual sugars, during fermentation in the fermentation vessel;
determining an RSI setpoint based on a rate of change of the measured residual sugar indicator parameter, such that the RSI setpoint corresponds to a maximum rate of change; and
automatically adapting the amount of sugar added to the fermentation vessel in a predetermined manner in response to the measured residual sugar indicator parameter RSI and the RSI setpoint, so as to achieve and maintain the RSI setpoint, whereby efficient co-fermentation of sugars to fermentation products is obtained.

2. The method of claim 1, further comprising the step of
in situ growing of the fermentation microorganisms in the fermentation vessel by means of the sugars added to the fermentation vessel, so as to maintain a steady-state population of fermentation microorganisms in the fermentation vessel, whereby further addition of microorganisms is not required, and wherein
the RSI setpoint corresponds to a steady state condition in the fermentation vessel.

3. The method of claim 1 or 2, wherein the RSI setpoint comprises a target interval of the RSI or a target interval of the RSI rate of change, and the step of automatically adapting in turn comprises the steps of:
comparing measured, and preferably processed, values of the residual sugar indicator parameter to the RSI setpoint; and
adjusting the amount of sugar added to the fermentation vessel in response to the comparison, so as to reach and stay within the RSI setpoint.

4. The method of any of previous claims, wherein the fermentation process is a fed-batch fermentation process comprising at least one batch phase, feed phase and end phase, respectively, and having continuous addition of fermentation media during the at least one feed phase.

5. The method of claim 4, wherein the fermentation process is a repeated fed-batch fermentation process with continuous addition of fermentation media during at least two feed phases.

6. The method of claim 4 or 5, wherein the respective feed phase or feed phases of the fermentation process are extended so as to comprise a substantial portion of the fed-batch cycle.

7. The method of any of claims 4-6, wherein
the step of determining an RSI setpoint is based on initial RSI measurements during the batch phase; and
the step of automatically adapting comprises automatically adapting, during the feed phase, the amount of sugar added to the fermentation vessel in a predetermined manner in response to RSI measurements during the feed phase and said RSI setpoint, so as to achieve and maintain said RSI setpoint.

8. The method of any of previous claims, further comprising the step of
secondary fermentation, in at least one secondary fermentation vessel (33-1; 33-2) arranged downstream of the fermentation vessel (31), to further increase the co-fermentation of sugars to fermentation products.

9. The method of any of previous claims, wherein the measuring step involves density measurements.

10. The method of any of previous claims, wherein the measuring step involves refractive index (RI) measurements and the residual sugar indicator parameter RSI comprises a refractive index (RI) parameter.

11. The method of any of previous claims, wherein the measuring step involves any of a combination of measurements selected from the group of: optical measurements within UV, visual or IR wavelengths; measurements of carbon dioxide (CO₂) generation; and direct measurements of sugar concentration.

12. The method of any of previous claims, comprising
addition, to the fermentation vessel (31), of at least two separate sugar streams associated with the respective different sugars; and
individual adjustment of the respective at least two separate sugar streams associated with the respective different sugars.

13. The method of any of previous claims, further comprising the step of
recirculation of fermentation microorganisms from a position downstream of the fermentation vessel (31) and back into the fermentation vessel.

14. The method of any of the previous claims, wherein the RSI setpoint is variable.

15. The method of any of the previous claims, wherein the RSI setpoint is changed during the operation.

## Patentansprüche

1. Verfahren zum Steuern eines mikrobiellen Fermentationsverfahrens umfassend Kofermentation von Zuckern aus lignocellulosehaltiger Biomasse zu Fermentationsprodukten mithilfe von Fermentations-Mikroorganismen, umfassend die Schritte:
kontinuierliches oder halbkontinuierliches Zugeben eines Fermentationsmediums, das wenigstens zwei verschiedene Zucker umfasst, zu einem Fermentationsgefäß (31);
Zuführen einer aktiven Anfangspopulation der Fermentations-Mikroorganismen in das Fermentationsgefäß;
Online-Messung eines Restzucker-Indikatorparameters RSI, welcher Parameter die Konzentration von Restzuckern während der Fermentation in dem Fermentationsgefäß direkt oder indirekt anzeigt;
Bestimmen eines RSI-Sollwerts auf der Grundlage einer Veränderungsrate des gemessenen Restzucker-Indikatorparameters, so dass der RSI-Sollwert einer höchsten Veränderungsrate entspricht; und
automatisches Anpassen der Menge an dem Fermentationsgefäß zugegebenem Zucker auf eine vorgegebene Weise in Antwort auf den gemessenen Restzucker-Indikatorparameter RSI und den RSI-Sollwert, um den RSI-Sollwert zu erreichen und aufrechtzuhalten, wodurch effiziente Kofermentation von Zuckern zu Fermentationsprodukten erhalten wird.

2. Verfahren gemäß Anspruch 1, ferner umfassend den Schritt
In-situ-Wachstum der Fermentations-Mikroorganismen in dem Fermentationsgefäß mithilfe der dem Fermentationsgefäß zugegebenen Zucker, um eine stationäre Population von Fermentations-Mikroorganismen in dem Fermentationsgefäß aufrechtzuhalten, wodurch weitere Zugabe von Mikroorganismen nicht erforderlich ist, und wobei
der RSI-Sollwert einem stationären Zustand in dem Fermentationsgefäß entspricht.

3. Verfahren gemäß Anspruch 1 oder 2, wobei der RSI-Sollwert ein Zielintervall des RSI oder ein Zielintervall der RSI-Veränderungsrate umfasst und der Schritt des automatischen Anpassens seinerseits die Schritte umfasst:
Vergleichen von gemessenen und vorzugsweise verarbeiteten Werten des Restzucker-Indikatorparameters mit dem RSI-Sollwert; und
Anpassen der Menge an dem Fermentationsgefäß zugegebenem Zucker in Antwort auf den Vergleich, um den RSI-Sollwert zu erreichen und darin zu bleiben.

4. Verfahren gemäß einem der vorstehenden Ansprüche, wobei das Fermentationsverfahren ein Fed-Batch-Fermentationsverfahren ist, umfassend jeweils wenigstens eine Batch-Phase, Zulaufphase und Endphase, und kontinuierliche Zugabe von Fermentationsmedium während der wenigstens einen Zulaufphase aufweist.

5. Verfahren gemäß Anspruch 4, wobei das Fermentationsverfahren ein wiederholtes Fed-Batch-Fermentationsverfahren mit kontinuierlicher Zugabe von Fermentationsmedium während wenigstens zwei Zulaufphasen ist.

6. Verfahren gemäß Anspruch 4 oder 5, wobei die entsprechende(n) Zulaufphase oder Zulaufphasen des Fermentationsverfahrens ausgedehnt ist/sind, um einen wesentlichen Anteil des Fed-Batch-Zyklus zu umfassen.

7. Verfahren gemäß einem der Ansprüche 4-6, wobei
der Schritt des Bestimmens eines RSI-Sollwerts auf der Grundlage von Anfangs-RSI-Messungen während der Batch-Phase steht; und
der Schritt des automatischen Anpassens automatisches Anpassen der Menge an dem Fermentationsgefäß zugegebenem Zucker während der Zulaufphase auf eine vorgegebene Weise in Antwort auf RSI-Messungen während der Zulaufphase und den RSI-Sollwert umfasst, um den RSI-Sollwert zu erreichen und aufrechtzuhalten.

8. Verfahren gemäß einem der vorstehenden Ansprüche, ferner umfassend den Schritt von
sekundärer Fermentation in wenigstens einem sekundären Fermentationsgefäß (33-1; 33-2), das stromabwärts bezogen auf das Fermentationsgefäß (31) angeordnet ist, um die Kofermentation von Zuckern zu Fermentationsprodukten weiter zu erhöhen.

9. Verfahren gemäß einem der vorstehenden Ansprüche, wobei der Messschritt Dichtemessungen umfasst.

10. Verfahren gemäß einem der vorstehenden Ansprüche, wobei der Messschritt Messungen des Brechungsindex (RI) umfasst und der Restzucker-Indikatorparameter RSI einen Brechungsindex(RI)-Parameter umfasst.

11. Verfahren gemäß einem der vorstehenden Ansprüche, wobei der Messschritt eine beliebige Kombination von Messungen ausgewählt aus der Gruppe von: optischen Messungen mit UV-, sichtbaren oder IR-Wellenlängen; Messungen von Kohlendioxid(CO₂)-Erzeugung; und direkten Messungen von Zuckerkonzentration umfasst.

12. Verfahren gemäß einem der vorstehenden Ansprüche, umfassend
Zugeben von wenigstens zwei getrennten Zuckerströmen, die mit entsprechenden verschiedenen Zuckern verbunden sind, zu dem Fermentationsgefäß (31); und
einzelnes Einstellen der entsprechenden wenigstens zwei getrennten Zuckerströme, die mit den entsprechenden verschiedenen Zuckern verbunden sind.

13. Verfahren gemäß einem der vorstehenden Ansprüche, ferner umfassend den Schritt
Rezirkulieren von Fermentations-Mikroorganismen von einer Position stromabwärts bezogen auf das Fermentationsgefäß (31) zurück in das Fermentationsgefäß.

14. Verfahren gemäß einem der vorstehenden Ansprüche, wobei der RSI-Sollwert variabel ist.

15. Verfahren gemäß einem der vorstehenden Ansprüche, wobei der RSI-Sollwert während des Betriebs verändert wird.

## Revendications

1. Procédé de commande d'un processus de fermentation microbienne impliquant la co-fermentation de sucres issus de la biomasse lignocellulosique en produits de fermentation au moyen de micro-organismes de fermentation, comprenant les étapes suivantes :
ajout continu ou semi-continu, dans une cuve de fermentation (31), d'un milieu de fermentation comprenant au moins deux sucres différents ;
fourniture d'une population active initiale de micro-organismes de fermentation dans la cuve de fermentation ;
mesure en ligne d'un paramètre indicateur de sucre résiduel RSI, lequel paramètre indique directement ou indirectement la concentration de sucres résiduels, au cours de la fermentation dans la cuve de fermentation ;
détermination d'un point de consigne RSI sur la base d'une vitesse de variation du paramètre indicateur de sucre résiduel mesuré, de sorte que le point de consigne RSI corresponde à une vitesse de variation maximale ; et
adaptation automatiquement de la quantité de sucre ajoutée à la cuve de fermentation d'une manière prédéterminée en réponse au paramètre indicateur de sucre résiduel mesuré RSI et au point de consigne RSI, de manière à atteindre et à maintenir le point de consigne RSI, ce qui permet d'obtenir une co-fermentation efficace des sucres en produits de fermentation.

2. Procédé selon la revendication 1, comprenant en outre l'étape de :
croissance in situ des micro-organismes de fermentation dans la cuve de fermentation au moyen des sucres ajoutés à la cuve de fermentation, de manière à maintenir une population stable de micro-organismes de fermentation dans la cuve de fermentation, de sorte qu'il n'est pas nécessaire d'ajouter d'autres micro-organismes, et
le point de consigne RSI correspondant à un état stable dans la cuve de fermentation.

3. Procédé selon la revendication 1 ou 2, le point de consigne RSI comprenant un intervalle cible du RSI ou un intervalle cible du taux de variation du RSI, et l'étape d'adaptation automatique comprenant à son tour les étapes de :
comparaison de valeurs mesurées, et de préférence traitées, du paramètre indicateur de sucre résiduel au point de consigne RSI ; et
ajustement de la quantité de sucre ajoutée à la cuve de fermentation en réponse à la comparaison, de manière à atteindre et à rester dans les limites du point de consigne RSI.

4. Procédé selon l'une quelconque des revendications précédentes, le processus de fermentation étant un processus de fermentation discontinu comprenant au moins une phase discontinue, une phase d'alimentation et une phase finale, respectivement, et ayant un ajout continu de milieu de fermentation pendant l'au moins une phase d'alimentation.

5. Procédé selon la revendication 4, le processus de fermentation étant un processus de fermentation discontinu répété avec ajout continu de milieu de fermentation pendant au moins deux phases d'alimentation.

6. Procédé selon la revendication 4 ou 5, la ou les phases d'alimentation respectives du processus de fermentation étant prolongées de manière à comprendre une partie substantielle du cycle discontinu.

7. Procédé selon l'une quelconque des revendications 4 à 6,
l'étape de détermination d'un point de consigne RSI étant basée sur des mesures RSI initiales pendant la phase discontinue ; et
l'étape d'adaptation automatique comprenant l'adaptation automatique, pendant la phase d'alimentation, de la quantité de sucre ajoutée à la cuve de fermentation d'une manière prédéterminée en réponse aux mesures RSI pendant la phase d'alimentation et audit point de consigne RSI, afin d'atteindre et de maintenir ledit point de consigne RSI.

8. Procédé selon l'une quelconque des revendications précédentes, comprenant en outre l'étape de
fermentation secondaire, dans au moins une cuve de fermentation secondaire (33-1 ; 33-2) disposée en aval de la cuve de fermentation (31), afin d'augmenter encore la co-fermentation des sucres en produits de fermentation.

9. Procédé selon l'une quelconque des revendications précédentes, l'étape de mesure comprenant des mesures de densité.

10. Procédé selon l'une quelconque des revendications précédentes, l'étape de mesure impliquant des mesures d'indice de réfraction (IR) et le paramètre indicateur de sucre résiduel RSI comprenant un paramètre d'indice de réfraction (IR).

11. Procédé selon l'une quelconque des revendications précédentes, l'étape de mesure impliquant une combinaison de mesures choisies dans le groupe des mesures optiques dans les longueurs d'onde UV, visuelles ou IR, des mesures de la génération de dioxyde de carbone (CO₂) et des mesures directes de la concentration en sucre.

12. Procédé selon l'une quelconque des revendications précédentes, comprenant :
l'ajout, dans la cuve de fermentation (31), d'au moins deux flux de sucre distincts associés aux différents sucres respectifs ; et
l'ajustement individuel des au moins deux flux de sucre séparés associés aux différents sucres respectifs.

13. Procédé selon l'une quelconque des revendications précédentes, comprenant en outre l'étape de
recirculation des micro-organismes de fermentation à partir d'une position en aval de la cuve de fermentation (31) et à nouveau dans la cuve de fermentation.

14. Procédé selon l'une quelconque des revendications précédentes, le point de consigne RSI étant variable.

15. Procédé selon l'une quelconque des revendications précédentes, le point de consigne RSI étant modifié au cours de l'opération.
